# EUROPÄISCHE PATENTSCHRIFT

(11) **EP 1 716 830 B1**
(45) Veröffentlichungstag und Bekanntmachung des Hinweises auf die Patenterteilung: **25.07.2007**
(21) Anmeldenummer: 05009126.3
(22) Anmeldetag: 26.04.2005
(51) Int. Cl.: A61F 13/15, A61L 15/22, B29C 47/88, B29C 59/04, B29C 55/00

(54) **Verfahren zur Herstellung einer Folienbahn**
Method for producing a web
Procédé pour la fabrication d'une bande

(43) Veröffentlichungstag der Anmeldung: 02.11.2006
(73) Patentinhaber: RKW AG Rheinische Kunststoffwerke, 67547 Worms (DE)
(72) Erfinder: Börmann, Ludwig, 83547 Babensham (DE); Schreiner, Günter, 83530 Schnaitsee (DE)
(74) Vertreter: Bublak, Wolfgang

(56) Entgegenhaltungen:
- EP-A- 0 256 885
- EP-A- 0 452 813
- EP-A- 0 768 168
- WO-A-95/32089
- US-A- 3 548 048
- US-A- 4 880 422
- US-A- 5 358 792
- US-B1- 6 232 402
- US-B1- 6 444 302

## Beschreibung

Die Erfindung betrifft ein Verfahren zur Herstellung einer Folienbahn, eine damit hergestellte Folienbahn sowie ihre Verwendung, speziell als Hygienefolie, z.B. Windelfolie oder Medikalfolie.

Derartige Hygienefolien werden vorzugsweise aus Polyolefinen hergestellt und besitzen üblicherweise eine Dicke von 5 bis 40 µm. Aufgrund ihres Einsatzbereichs müssen sie flüssigkeitsdicht sein. An z.B. Folien für Hygieneprodukte wird hierbei eine ganze Reihe von weiteren Anforderungen gestellt. Hierzu gehören auch die haptischen Eigenschaften der Folie, also z. B. Weichheit, Geschmeidigkeit, raschelarmes Verhalten und textiler Griff.

Folien im Hygienebereich sollen einen weichen, stoffähnlichen Griff besitzen. Insbesondere bei ihrer Verwendung für Inkontinenzprodukte soll die Geräuschentwicklung möglichst gering sein, d.h. die Folien sollen raschelarm sein. In Verbindung mit einem geringen Glanzgrad ergibt dies eine sehr textile Folie, was im Hygienebereich wichtig ist.

In den letzten Jahren sind die in Windeln und Inkontinenzprodukten enthaltenen Saugkörper ständig dünner geworden, was insbesondere durch die Verwendung von Superabsorber-Polymeren ermöglicht wurde. Diese Superabsorber-Polymere finden in Form grobkörniger Pulver Verwendung, und die Hygienefolien müssen eine derartige Festigkeit besitzen, dass ein Durchstoßen der Folie durch die einzelnen Körner, z.B. bei Belastung durch Hinsetzen oder sonstige Bewegung des Trägers, mit Sicherheit vermieden wird. Eine Bildung von Löchern ("*Pinholes*") durch Superabsorber-Polymere und ein Platzen der Fertigfolienprodukte in den Verpackungseinheiten muss vermieden werden. Diese Anforderung an die Durchstoßfestigkeit hat bis heute dem Trend zu dünneren Hygienefolien Grenzen gesetzt.

Eine weitere Anforderung an Hygienefolien besteht in einer Mindestzugfestigkeit, die für eine Verarbeitung der Folienbahnen auf den schnell laufenden Maschinen (Konverter) der Hersteller von z.B. Windelfolien und Damenbinden erforderlich ist. Diese Mindestzugfestigkeit wird für 5%, 10 % oder 25 % Dehnung in Maschinenrichtung (*md*) oder Querrichtung (*cd*) angegeben. Derzeit soll die Zugfestigkeit bei 5 % Dehnung (5%-Modul) in Maschinenrichtung wenigstens 3 N/inch betragen. Darüber hinaus sollen Folien für Hygieneanwendungen eine Querreißfestigkeit von wenigstens 3.94 N/cm (10 N/inch) aufweisen.

Aus der DE-A-33 26 056 ist ein Verfahren zum Herstellen thermoplastischer Folien bekannt, bei dem eine Masse aus LLDPE (lineares Polyethylen geringer Dichte) im Schlitzdüsenverfahren in Form einer Bahn extrudiert wird, wobei die Masse auf einer Temperatur oberhalb ihrer Schmelztemperatur gehalten wird. Die Bahn wird entlang eines in seiner Länge definierten Zugspaltes von der Schlitzdüse zu einer Zugwalze geführt und anschließend mittels der Zugwalze zu einer Folie ausgezogen. Dabei wird die Oberfläche der Zugwalze auf einer Temperatur gehalten, die eine Erstarrung/Kristallisation der auf ihr befindlichen Folie bewirkt. Beschrieben sind Folien mit einer Dicke von 25 µm.

Die EP-A-0 616 880 beschreibt ein Verfahren zur Herstellung einer für Verpackungsmaterialien geeigneten Folie aus einem thermoplastischen Harz, bei dem Foliendicken zwischen 100 und 2000 µm erhalten werden. Bei diesem Verfahren wird eine thermoplastische Ausgangsfolie auf eine Temperatur zwischen ihrem Erweichungspunkt und ihrem Schmelzpunkt erwärmt und anschließend gekühlt.

Aus der EP-A-0 768 168 ist ein Verfahren zur Herstellung einer im Hygienebereich einsetzbaren Folienbahn bekannt, bei der eine Ausgangsfolienbahn aus thermoplastischem Polymermaterial bis zum schmelzflüssigen Zustand des Polymermaterials erwärmt und danach durch einen gekühlten Walzenspalt geführt wird.

Die EP-A-0 256 885 offenbart Unterlageschichten ("*Backsheets*") für Windeln, die im Wesentlichen aus Polyethylen geringer Dichte und 10 bis 20 Gew.-% Polypropylen bestehen. Durch die Zugabe des Polypropylens sollen hierbei unter anderem die Hafteigenschaften gegenüber dem Band zum Verschließen der Windel ("*tape adhesion*") verbessert werden. Die Dicke der beschriebenen Folien liegt im Bereich von 25 bis 38 µm (1,0 bis 1,5 mil). Aus der GB-A-2 152 515 sind Folien aus Polymergemischen bekannt, bei denen Polyethylen (LLDPE, LDPE) 2 bis 15 Gew.-% Polypropylen zur Erhöhung der Steifigkeit zugesetzt werden. In der GB-A-2 152 516 des gleichen Anmelders wird berichtet, dass Folien aus LLDPE mit einem Zusatz an Polypropylen zwar erhöhte Steifigkeit gegenüber Folien aus LLDPE allein aufweisen, derartige Folien jedoch eine "katastrophale Abnahme der Durchstoß- und Reißfestigkeit, insbesondere in Maschinenrichtung (md) zeigen". In der GB-A-2 152 516 werden deshalb bis zu 2 Gew.-% spezielle Elastomere zugesetzt. Diese Elastomere sind jedoch einerseits äußerst schwer zu extrudieren und andererseits sehr teuer.

EP-A-0 452 813 betrifft ein zweistufiges Verfahren zum Kaltformen von Polyethylen-Polypropylen-Copolymeren zur Herstellung von Formteilen. Die US-A-3 548 048 ist ebenfalls auf ein zweistufiges Verfahren gerichtet, bei dem polymere Gegenstände hergestellt werden, die gegenüber Fibrillierung (Faserbildung) widerstandsfähig sind. WO 95/32 089 offenbart ein Verfahren zur Erhöhung des Kristallitschmelzpunkts eines Polymers, beispielsweise Polypropylen, durch Erwärmen des Polymers in der Nähe seines Kristallitschmelzpunkts (Tₘ), wodurch ein Teil des Polymers geschmolzen wird, und anschließendes Abkühlen.

Wie Eingangs erwähnt, sind dem Trend nach immer dünneren Hygienefolien dadurch Grenzen gesetzt, dass für die Verarbeitung der Folienbahnen auf den schnell laufenden Maschinen der Verarbeiter Mindestanforderungen an die mechanischen Eigenschaften erfüllt sein müssen, da andernfalls eine fachgerechte Verarbeitung nicht mehr gewährleistet ist oder die Verarbeitungsprodukte mit Eigenschaften behaftet sind, die von den Abnehmern nicht akzeptiert werden. Mindestanforderungen bestehen z. B. bezüglich der Zugfestigkeit, der Zugfestigkeit bei 5 %, 10 % und 25 % Dehnung sowie der Reißdehnung, jeweils in Maschinenrichtung (md) und Querrichtung (cd), sowie der Durchstoßfestigkeit. Weitere Mindestanforderungen bestehen an die Weichheit bzw. Geschmeidigkeit der Folie, die einen möglichst textilen Griff aufweisen soll sowie an das Raschelverhalten, d. h. die Folie soll möglichst raschelarm sein. Bei den konventionellen

Verfahren zur Herstellung von Folienbahnen für den Hygienebereich sind die heutigen Mindestanforderungen bis herab zur Folienstärken von etwa 25 µm erfüllt. Dem gegenüber hat das Verfahren gemäß EP-A-0 768 168 bereits eine Verbesserung gebracht, so dass die Foliendicke bis auf 23 µm und in manchen Fällen auch noch weiter verringert werden konnte. Die Aufgabe der Erfindung bestand nun darin, die gewünschten Eigenschaften der Folien weiter zu verbessern, so dass insbesondere auch bei Foliendicken von 20 µm oder sogar darunter diese Mindesteigenschaften erfüllt sind.

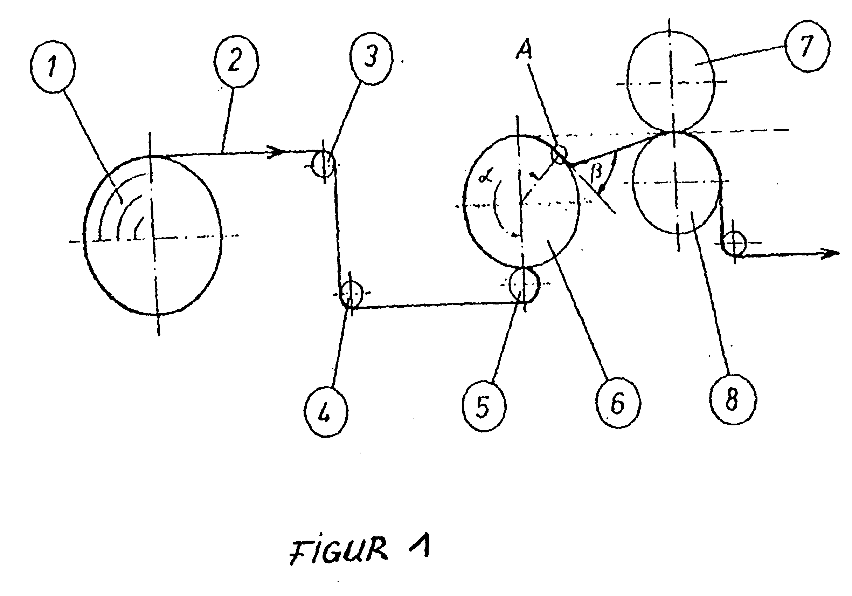

Gegenstand der Erfindung ist somit ein Verfahren zur Herstellung einer Folienbahn, wobei eine Ausgangsfolienbahn aus thermoplastischem Polymermaterial mit einer Polyethylen-Matrix, in der 1 bis 70 Gewichtsteile Polypropylen, bezogen auf 100 Gewichtsteile Polyethylen-Matrix, enthalten sind, nach ihrer Erwärmung durch einen gekühlten Walzenspalt geführt wird, dass dadurch gekennzeichnet ist, das man die Erwärmung der Ausgangsfolienbahn bis zum schmelzflüssigen Zustand des Polyethylen-Matrixmaterials, jedoch nicht bis zum schmelzflüssigen Zustand des Polypropylens führt. Weiterhin betrifft die Erfindung die mit diesem Verfahren hergestellten Folienbahnen sowie deren Verwendung, insbesondere im Hygiene- und Medikalbereich. Bevorzugte Ausführungsformen der Erfindung sind in der nachfolgenden Beschreibung, der Figur, dem Beispiel und den Unteransprüchen beschrieben.

Das Verfahren der Erfindung löst die gestellte Aufgabe, d. h. es ermöglicht die Herstellung von kommerziell verwertbaren Folienbahnen mit den gewünschten Eigenschaften, die eine verringerte Foliendicke von unter 20 µm, z. B. 18 oder 16 oder auch nur 15 µm aufweisen. Ein weiterer Vorteil der nach dem Verfahren der Erfindung erhaltenen Folienbahnen besteht in einer verbesserten Thermostabilität. Bei der Verwendung der Folienbahnen als sogenannte Backsheets im Hygienebereich wird die Innenausstattung von z. B. Babywindeln oder Inkontinenzartikeln mittels Hotmelt-Klebersystemen aufgebracht, die bei Temperaturen im Bereich von 140 bis 160 °C aufgebracht werden. Hierbei dürfen die gewünschten Eigenschaften der Backsheets nicht verloren gehen, was bei den herkömmlichen Back sheets nur bei größeren Foliendicken möglich ist.

Bekanntlich besitzen Polymere keinen scharf definierten Schmelzpunkt, sondern einen Schmelzbereich, wobei sich jedoch den kristallinen Bereichen eines Polymeren ein Kristallitschmelzpunkt zuordnen lässt. Dieser Kristallitschmelzpunkt liegt stets höher als der Schmelzpunkt (Bereich) der nicht-kristallinen Bestandteile. In jedem Fall ist der schmelzflüssige Zustand dadurch beschrieben, dass der Schubmodul gegen Null geht und - im Falle von Polymeren mit kristallinen Bereichen - letztere nicht mehr nachweisbar sind. Der Schubmodul kann beispielsweise nach ISO 6721-1 & 2 bestimmt werden. Bei der vorliegenden Erfindung wird die Ausgangsfolienbahn auf eine Temperatur erwärmt, bei der für das Polyethylen-Matrixmaterial der Schubmodul gegen Null geht und keine kristallinen Bereiche mehr nachweisbar sind. Hingegen geht bei dieser Temperatur für das Polypropylen der Schubmodul nicht gegen Null und es sind noch kristalline Bereiche nachweisbar. Der Schubmodul des gesamten Polymermaterials der Ausgangsfolienbahn geht somit nicht gegen Null und es sind noch kristalline Bereiche des Polypropylens nachweisbar.

Die Polyethylen-Matrix der Folienbahn besteht hauptsächlich aus Ethylenpolymeren, wobei sowohl Ethylenhomopolymere als auch Ethylencopolymere mit Ethylen als Hauptcomonomerem geeignet sind. Geeignete Ethylenhomopolymere sind LDPE *(Low Density Polyethylene),* LLDPE (*Linear Low Density Polyethylene*), MDPE (*Medium Density Polyethylene*) und HDPE (*High Density Polyethylene*). Bevorzugte Comonomere für Ethylencopolymere sind andere Olefine mit Ausnahme von Propylen, z. B. Buten, Hexen oder Octen. Vorzugsweise liegt hierbei der Comonomergehalt unter 20 Gew.-%, insbesondere unter 15 Gew.-%.

In einer bevorzugten Ausführungsform besteht die Polyethylen-Matrix ausschließlich aus Ethylenhomopolymeren, z. B. Gemischen aus LDPE und LLDPE, die jeweils in Mengen von 10 bis 90 Gew.-% enthalten sein können. Ein spezielles Beispiel ist eine Polyethylenmatrix aus 60 Gew.-% LDPE und 40 Gew.-% LLDPE.

Neben den Ethylenhomo- oder -copolymeren kann die Polyethylenmatrix auch andere thermoplastische Polymere enthalten, wobei jedoch darauf zu achten ist, dass hierdurch die Temperatur, bei der sich das gesamte Polymermatrixmaterial in schmelzflüssigem Zustand befindet, nicht zu nahe an die Temperatur heranrückt, bei der sich das Polypropylen in schmelzflüssigem Zustand befinden würde, siehe hierzu weiter unten.

In der Ausgangsfolienbahn sind in der Polyethylen-Matrix 1 bis 70 Gewichtsteile Polypropylen, bezogen auf 100 Gewichsteile Polyetylen-Matrix, enthalten. Vorzugsweise beträgt die Menge an Polypropylen 5 bis 65 Gewichtsteile, insbesondere 5 bis 45 Gewichtsteile und besonders bevorzugt 10 bis 40 Gewichtsteile, jeweils bezogen auf 100 Gewichtsteile der Polyethylen-Matrix. Spezielle Beispiele für in der Polyethylen-Matrix enthaltene Polypropylenmengen sind 14, 16, 18, 25 oder 28 Gewichtsteile Polypropylen.

Die Bezeichnung Polypropylen umfasst hier sowohl Propylenhomo- als auch -copolymere mit Propylen als Hauptcomonomerem. Bei den Propylencopolymeren ist hierbei der Anteil des Comonomeren, d. h. Nicht-Propylen, erfindungsgemäß der Polyethylen-Matrix zuzurechnen. Geeignete Comonomere für Propylencopolymere sind andere Olefine, vorzugsweise Ethylen. Bei den Propylenethylen-Copolymeren beträgt der Ethylenanteil vorzugsweise 2 bis 30 Gew.-%, besonders bevorzugt 2 bis 20 Gew.-% und insbesondere 2 bis 15 Gew.-%, wobei in der Praxis bei einem Ethylengehalt von 3 bis 12 Gew.-% sehr gute Ergebnisse erhalten werden. Diese Zahlenwerte gelten auch für die anderen Olefine. Copolymere von Polypropylen mit Ethylen sind als Handelsprodukte, beispielsweise für die Herstellung von Blas- und/oder *Cast*-Folien, erhältlich.

In einer besonderen Ausführungsform wird eine Ausgangsfolienbahn folgender Zusammensetzung verwendet: Polyethylen-Matrix aus 40 Gew.-% eines Ethylenoctencopolymeren mit 5-10 Gew.-% Octenanteil , Rest LDPE; 5 bis 45 Gewichtsteile Propylenethylen-Copolymeres mit 3 bis 12 Gew.-% Ethylen, bezogen auf 100 Gewichtsteile Polyethylen-Matrix.

Bei dem Verfahren der Erfindung wird die Erwärmung der Ausgangsfolienbahn bis zum schmelzflüssigen Zustand des Polyethylenmatrixmaterials, jedoch nicht bis zum schmelzflüssigen Zustand des Polypropylens geführt. Es ist vorstehend bereits ausgeführt worden, das Polymere keinen definierten Schmelzpunkt sondern einen Schmelzbereich aufweisen, wobei sich jedoch den kristallinen Bereichen eines Polymeren ein Kristallitschmelzpunkt zuordnen lässt, wobei wiederum dieser Kristallitschmelzpunkt stets oberhalb des Schmelzbereiches der nicht-kristallinen Bereiche liegt. Nachfolgend sind für einige Polyethylenmatrixmaterialien und darin enthaltenes Polypropylen die Schmelzbereiche angegeben.
LDPE: 112 - 114°C;
LLDPE: 119 - 125°C;
Propylenhomopolymere: 155 - 165°C;
Propylenethylen-Copolymere: 130 - 162°C, bei sehr wenig Ethylen auch höher.

Zur Erreichung des wesentlichen Verfahrensmerkmals der Erfindung, d. h. Erwärmung der Ausgangsfolienbahn bis zum schmelzflüssigen Zustand der Polyethylenmatrix, jedoch nicht bis zum schmelzflüssigen Zustand des Polypropylens, steht also ein ausreichender Temperaturabstand zur Verfügung, und, sofern die vorgenannte Bedingung erfüllt ist, spielt der speziell gewählte Temperaturabstand keine besondere Rolle, vielmehr wird dieser durch praktische Überlegungen bezüglich Sicherheit der Verfahrensdurchführung oder wirtschaftliche Überlegungen bestimmt. Wenn z. B. bei einer bestimmten Verfahrenstemperatur die Polymermatrix einwandfrei aufgeschmolzen ist, bringt eine weitere Temperaturerhöhung keine besseren Ergebnisse. Zudem steigt der Wärmeverbrauch und man kommt gegebenenfalls zu nahe an den Schmelzbereich des Polypropylens heran, so dass das Verfahren schwieriger durchzuführen ist. Vorzugsweise wird deshalb das Verfahren der Erfindung so durchgeführt, dass die Erwärmung der Ausgangsfolienbahn bis auf eine Temperatur nicht höher als 10 bis 15 °C unterhalb des Kristallitschmelzpunkts des Polypropylens erfolgt.

Die beim Verfahren der Erfindung eingesetzten Ausgangsfolienbahnen können eingefärbt sein, z. B. weiß mit Titandioxid. Weiterhin können die Ausgangsfolienbahnen übliche Zusatzstoffe und Verarbeitungshilfsmittel enthalten.

In den beim Verfahren der Erfindung eingesetzten Ausgangsfolienbahnen ist das Polypropylen in einer Polyethylen-Matrix enthalten. Bei der Herstellung der Ausgangsfolienbahn, z. B. nach dem Schlitzdüsenverfahren oder Blasverfahren, muss das Gemisch aus Polyethylenmatrixmaterial und Polypropylen im Extruder auf jeden Fall bis deutlich über die Schmelzflusstemperatur aller Polymerbestandteile erhitzt werden, z. B. bis über 200 °C, wodurch eine homogene Durchmischung in der Schmelze erreicht wird.

Die Dicke der Ausgangsfolienbahn liegt z. B. im Bereich von 5 bis 40 µm, vorzugsweise 10 bis 30 µm und besonders bevorzugt 12 bis 25 µm. Das erfindungsgemäße Verfahren ermöglicht die Herstellung von Folien mit geringer Foliendicke von z. B. 20, 18, 16 oder auch nur 15 µm, die gleichwohl die an Hygienefolien gestellten Anforderungen, insbesondere bezüglich Weichheit, Raschelverhalten und Durchstoßfestigkeit erfüllen.

Erfindungsgemäß kann die Erwärmung der Ausgangsfolienbahn auf verschiedene Weise erfolgen. Vorzugsweise erfolgt jedoch die Erwärmung mittels einer oder mehrerer Kontaktwalzen/-Heizwalzen, die mittels eines Wärmeträgers auf die vorgegebene Temperatur erhitzt werden.

Um zu gewährleisten, dass die Ausgangsfolienbahn die Walzentemperatur tatsächlich erreicht, ist für eine ausreichende Verweilzeit der Ausgangsfolienbahn auf der Heizwalzenoberfläche zu sorgen. Dies kann auch durch Vergrößerung des Umschlingungswinkels α (vgl. die Figur), durch Vergrößerung des Walzendurchmessers und/oder Verringerung der Folienbahngeschwindigkeit nach Maßgabe der Foliendicke erfolgen.

In einer anderen Ausführungsform erfolgt die Erwärmung der Ausgangsfolienbahn mittels Strahlungswärme, entweder ausschließlich oder zur Unterstützung der Erwärmung mittels Heizwalzen.

Eine Schwierigkeit bei der Verarbeitung der Folienbahn im schmelzflüssigen Zustand der Polyethylen-Matrix mittels Heizwalzen besteht darin, dass die Folienbahn sehr viel stärker an den Heizwalzen haftet als bei einer herkömmlichen Verarbeitung unterhalb der Temperatur des schmelzflüssigen Zustands. Dies erfordert eine erhöhte Ablösekraft. Die erhöhte Haftung der Folienbahn auf der Heizwalze hat auch eine Verschiebung des Ablösepunktes in Drehrichtung der Heizwalze zur Folge, was wiederum eine Vergrößerung des Ablösewinkels β (vgl. die Figur) bedeutet. Die Erhöhung der Ablösekraft kann hierbei soweit gehen, dass die Folienbahn abreißt. Andererseits kann die erhöhte Haftung dazu führen, dass sich die teilweise schmelzflüssige Folienbahn überhaupt nicht mehr von der Heizwalze ablösen lässt, mit der Walze umläuft, und auf diese Weise die Produktion zum Erliegen kommt. Aus diesem Grund wird erfindungsgemäß vorzugsweise eine Heizwalze mit modifizierter Oberfläche verwendet, die eine verringerte Haftung gegenüber der teilweise schmelzflüssigen Folienbahn aufweist. Vorzugsweise wird hierzu eine PTFE (Polytetrafluorethylen)-beschichtete Heizwalze verwendet. Auch andere antihaftende Oberflächen sind geeignet.

Infolge des teilweise schmelzflüssigen Zustandes der Folienbahn auf der Heizwalze kommt es zu einer Verschleppung der Ablösung der Folienbahn in Drehrichtung der Heizwalze. Das Ausmaß der Verschleppung ist von den Verfahrensparametern und dem Folienmaterial abhängig und wird durch den Ablösewinkel β definiert. Der kleinste Winkelwert (0°) ist durch die vom Kühl-Walzenspalt an die Heizwalze gelegte Tangente (in der Figur gestrichelt gezeichnet) gegeben, während der Winkel nach größeren Werten im Prinzip nur durch den Auflagepunkt der Ausgangsfolie an die Heizwalze begrenzt ist. In der praktischen Durchführung des Verfahrens sind jedoch Ablösewinkel β von deutlich über 90° unzweckmäßig, weil es dann zu einer Schlaufen- bzw. Sackbildung der teilweise schmelzflüssigern Folienbahn an der Heizwalze kommen kann.

Erfindungsgemäß wird die Ausgangsfolienbahn nach ihrer Erwärmung durch einen gekühlten Walzenspalt geführt. Die den Walzenspalt bildenden Walzen sind so gekühlt, z.B. mit Wasser in einem Temperaturbereich von 5 bis 20°C, dass eine rasche Abkühlung der Folienbahn auf eine Temperatur unterhalb des Kristallitschmelzpunktes des Polyethylen-Matrixmaterials, vorzugsweise 80 °C oder darunter, gewährleistet ist. Der Abstand zwischen der letzten Heizwalze und dem Kühl-Walzenspalt sollte hierbei wegen möglicher Wärmeverluste nicht zu groß sein. Für einen Mindestabstand ist ohnehin durch die Abmessungen der Walzen eine Grenze gesetzt. Bei dem Kühl-Walzenspalt kann es sich z.B. um einen Glattwalzenspalt handeln. Im Fall von Hygienefolien wird der Walzenspalt jedoch vorzugsweise von einem Walzenpaar mit Strukturwalze gebildet, wodurch die Folienbahn eine strukturierte Oberfläche erhält.

Die Folienbahngeschwindigkeiten bewegen sich in üblichem Rahmen, z.B. im Bereich von 50 bis 500 m/min, abhängig von den Folienparametern und den anderen Verfahrensbedingungen.

Die für die Ablösung der Folienbahn von der Heizwalze erforderliche Kraft wird von einer Vorspannung aufgebracht, die durch eine gegenüber der Bahngeschwindigkeit der Heizwalze erhöhte Bahngeschwindigkeit (Voreilung) im Kühl-Walzenspalt erreicht wird. Zur Aufbringung der Ablösekraft sind wenige Prozent Voreilung ausreichend, z.B. 0,5 bis 5%. Die Voreilung kann auch sehr viel höher gewählt werden, z.B. wenn eine Verringerung der Foliendicke erfolgen soll. Zum Beispiel erfolgt unter Anwendung einer Voreilung von 30% eine Verringerung der Foliendicke von 30 auf 20 µm.

Die Ausgangsfolienbahnen für die Durchführung des Verfahrens der Erfindung können in beliebiger Weise hergestellt werden, vorzugsweise werden sie nach dem Schlitzdüsenverfahren, bei dem eine Folie durch eine Breitschlitzdüse extrudiert wird, oder dem Blasverfahren hergestellt, wobei das Blasverfahren bevorzugt ist. Beim dem Blas- und Schlitzdüsenverfahren werden die Folien in bekannter Weise durch Extrusion erzeugt, wobei vorzugsweise auf gutes Vermischen im Extruder geachtet wird. Vorzugsweise finden solche Folienbahnen Verwendung, die bei ihrer Herstellung einer Verstreckung in Querrichtung unterworfen worden sind. Hierbei sind wiederum Blasfolienbahnen bevorzugt. Das Verfahren der Erfindung wird vorzugsweise mit einschichtigen Folienbahnen durchgeführt, es können jedoch auch mehrschichtige Folienbahnen verwendet werden.

Die Figur zeigt eine bevorzugte Ausführungsform zur Durchführung des erfindungsgemäßen Verfahrens. Von einer Rolle 1 läuft eine Ausgangsfolienbahn 2 über Umlenkwalzen 3 und 4 und eine Anpresswalze 5 auf eine Heizwalze 6. Bei der Heizwalze 6 handelt es sich z.B. um eine antihaftend beschichtete Stahlwalze, die mittels Wärmezufuhr auf die gewünschte Oberflächentemperatur erhitzt wird. Die Folienbahn läuft dann auf der Heizwalze 6 und wird dabei erfindungsgemäß erwärmt. Bei dem Umschlingungswinkel α handelt es sich um denjenigen Winkel, der gebildet wird vom ersten Berührungspunkt der Ausgangsfolienbahn 2 mit der Heizwalze 6 bis zu demjenigen Punkt in Drehrichtung der Heizwalze 6 gesehen, wo die Ablösung der Folienbahn von der Heizwalze erfolgt. Von der Heizwalze 6 läuft die Folienbahn unter einem Ablösewinkel β (Ablösepunkt A) in einen Kühl-Walzenspalt, der vom Walzenpaar 7 und 8 gebildet wird. Die Walze 8 ist vorzugsweise als Strukturwalze ausgebildet, wodurch die Folienbahn eine strukturierte Oberfläche erhält. Das Walzenpaar 7/8 ist vorzugsweise wassergekühlt. Die den Spalt bildenden Walzen 7 und 8 können so angetrieben werden, dass gegenüber der Bahngeschwindigkeit der Heizwalze 6 eine Voreilung besteht, was eine Verringerung der Folienbahndicke zur Folge hat. Nach dem Walzenpaar 7/8 wird die Folie abgenommen.

Die Erfindung ermöglicht die Herstellung von Folien, die die im Hygienebereich geforderten mechanischen und haptischen Eigenschaften bei gleichzeitig geringer Foliendicke in sich vereinigen. Es ist deshalb eine gute Verarbeitbarkeit zu den Fertigprodukten, z. B. Windeln, auf den herkömmlichen Konvertern möglich, und eine hohe Sicherheit gegen Einreißen oder Abreißen der Fertigprodukte sowie gegen Bildung von pinholes gewährleistet. Hierbei ist überraschend, dass trotz des Gehalts an Polypropylen eine geschmeidige, raschelarme Folie mit sympathischem textilen Griff aber gleichwohl hervorragenden mechanischen Eigenschaften erhalten wird.

Die Erfindung wird anhand des nachfolgenden Beispiels erläutert.

### BEISPIEL

Eine Ausgangsfolienbahn würde nach dem Blasverfahren mit einer Rezeptur gemäß Tabelle I hergestellt.

**TABELLE I**

| Folienrezeptur | | | | |
|---|---|---|---|---|
| Menge Gew.-Teile | Bestandteil | Dichte, g/cm³ | Kristallitschmelzpunkt, °C | Schmelzindex, g/10 min²⁾ |
| 59 | LDPE | 0,922-0,924 | 113 | 0,60-0,90 |
| 41 | LLDPE-Octen | 0,930 | 124 | 1,0 |
| 30 | Polypropylen¹⁾ | 0,90 | 162 | 0,4 |
| 5 | TiO₂ - Weiß-konzentrat | 1,69 | - | - |

| | | | | |
|---|---|---|---|---|
| ¹ *Propylenethylen-Copolymeres mit 10 Gew.-% Ethylen* ² 190°C/2,16 kg für LDPE und LLDPE und 230°C/2,16 kg für Polypropylen | | | | |

Die Bedingungen beim Blasen des Folienschlauches sind aus nachstehender Tabelle II ersichtlich.

**TABELLE II**

| Blasbedingungen | |
|---|---|
| Ringdüse | 600 mm Durchmesser |
| Düsenspalt | 1 mm |
| Schlauchdurchmesser | 1590 mm |
| Foliendicke | 16 µm |
| Extrudertemperatur | 240°C |

Der erhaltene Folienschlauch wurde in Längsrichtung aufgeschnitten und auf zwei Rollen aufgewickelt. Die Folienbreite betrug 2,5 m.

Diese Ausgangsfolienbahn wurde dem in der Figur gezeigten Verfahren wie folgt unterworfen. Nach dem Abziehen der Ausgangsfolienbahn 2 von der Rolle 1 läuft diese über die Umlenkwalzen 3, 4 und die Anpresswalze 5 auf die Heizwalze 6. Bei der Heizwalze 6 handelt es sich um eine antihaftend beschichtete Stahlwalze, die mittels Wärmezufuhr auf eine Oberflächentemperatur von 130°C erhitzt wird. Die Heizwalze 6 wird mit einer Bahngeschwindigkeit von 260 m/min angetrieben. Von der Heizwalze 6 läuft die Folienbahn in den von dem Walzenpaar 7/8 gebildeten Kühl-Walzenspalt. Die Walze 8 ist als Strukturwalze ausgebildet. Das Walzenpaar 7/8 ist wassergekühlt (15°C). Die den Spalt bildenden Walzen 7/8 sind so angetrieben, dass gegenüber der Bahngeschwindigkeit der Heizwalze 6 von 260 m/min eine Voreilung von 5% (13 m/min) besteht. Diese Voreilung hat eine Verringerung der Folienbahndicke von 16 auf 15 µm zur Folge. Hierbei bildet sich ein Ablösewinkel β ≤ 90° aus. Der Umschlingungswinkel α beträgt etwa 200°.

Die Messwerte dieser Folie (A) sind in Tabelle III mit den Messwerten einer nach dem Verfahren der EP-A-0 768 168 erhaltenen Folie (B) verglichen.

**Tabelle III**

| Messgröße | | Messnorm | Dimensionen | Folie A | Folie B |
|---|---|---|---|---|---|
| Dicke | | | µm | 15 | 15 |
| Prägehöhe | | EN ISO | µm | 31 | 31 |
| | | 2286-3 | | | |

| Zugfestigkeit bei | | EN ISO 527 | (N/inch) | | |
|---|---|---|---|---|---|
| Dehnung von | | | N/cm | | |
| 5% | md* | | | (3,5) 1,38 | (2,0) 0,79 |
| 10% | | | | (4,8) 1,89 | (3,2) 1,26 |
| 25% | | | | (7,0) 2,76 | (5,5)2,17 |
| Zugfestigkeit bei | | EN ISO 527 | (N/inch) | | |
| Dehnung von | | | N/cm | | |
| 5% | cd* | | | (3,5) 1,38 | (2,0) 0,79 |
| 10% | | | | (4,2) 1,65 | (2,3) 0,91 |
| 25% | | | | (4,3) 1,69 | (2,5) 0,98 |
| Reißfestigkeit | md | EN ISO 527 | (N/inch) | (19,0)7,48 | (14,0)5,51 |
| | cd | | N/cm | (13,0)5,12 | (9,0) 3,54 |
| Reißdehnung | md | EN ISO 527 | % | 300 | 270 |
| | cd | | | 700 | 700 |
| Glanz | | DIN 67530 | | 4,0 | 4,0 |
| Durchstoßfestigkeit | | Eigennorm, | mm | 500 | 500 |
| | | speziell für | | | |
| | | Windeln | | | |

| | | | | | |
|---|---|---|---|---|---|
| *) md = in Maschinenrichtung cd = quer zur Maschinenrichtung | | | | | |

Die Folien A und B zeigen nach der Beurteilung von Testpersonen den gleichen sympathischen textilähnlichen Griff und das gleiche Raschelverhalten. Auch die Durchstoßfestigkeit ist bei beiden Folien gleich. Hingegen zeigt die Folie A gegenüber der Folie B bezüglich Zugfestigkeit und Reißfestigkeit dramatische Verbesserungen.

## Patentansprüche

1. Verfahren zur Herstellung einer Folienbahn, wobei eine Ausgangsfolienbahn aus thermoplastischem Polymermaterial mit einer Polyethylen-Matrix, in der 1 bis 70 Gewichtsteile Polypropylen, bezogen auf 100 Gewichtsteile Polyethylen-Matrix, enthalten sind, nach ihrer Erwärmung durch einen gekühlten Walzenspalt geführt wird, **dadurch gekennzeichnet, dass** man die Erwärmung der Ausgangsfolienbahn bis zum schmelzflüssigen Zustand des Polyethylen-Matrixmaterials, jedoch nicht bis zum schmelzflüssigen Zustand des Polypropylens führt.

2. Verfahren nach Anspruch 1, **dadurch gekennzeichnet, dass** man eine Ausgangsfolienbahn mit 5 bis 45 Gewichtsteilen Polypropylen, bezogen auf 100 Gewichtsteile Polyethylen-Matrix, verwendet.

3. Verfahren nach Anspruch 2, **dadurch gekennzeichnet, dass** man eine Ausgangsfolienbahn mit 10 bis 40 Gewichtsteilen Polypropylen, bezogen auf 100 Gewichtsteile Polyethylen-Matrix, verwendet.

4. Verfahren nach einem der vorhergehenden Ansprüche, **dadurch gekennzeichnet, dass** man eine Ausgangsfolienbahn mit einer Polyethylenmatrix aus 60 Gew.-% LDPE und 40 Gew.-% LLDPE verwendet.

5. Verfahren nach Anspruch 3, **dadurch gekennzeichnet, dass** man eine Ausgangsfolienbahn mit einer Polyethylenmatrix aus 60 Gew.-% LDPE und 40 Gew.-% LLDPE-Octen mit einem Octengehalt von 5-10 Gew.-%, und einem Gehalt an Propylenethylen-Copolymerem, das 3 bis 12 Gew.-% Ethylen als Comonomeres enthält, von 30 Gewichtsteilen, bezogen auf 100 Gewichtsteile Polyethylen-Matrix, verwendet.

6. Verfahren nach einem der vorhergehenden Ansprüche, **dadurch gekennzeichnet, dass** man eine Ausgangsfolienbahn mit einer Dicke von 10 bis 30 µm verwendet.

7. Verfahren nach einem der vorhergehenden Ansprüche, **dadurch gekennzeichnet, dass** man die Erwärmung der Ausgangsfolienbahn mittels einer oder mehrerer Heizwalzen durchführt.

8. Verfahren nach Anspruch 7, **dadurch gekennzeichnet, dass** man Heizwalzen mit verringerter Haftneigung gegenüber der Folienbahn mit schmelzflüssiger Polyethylen-Matrix verwendet.

9. Verfahren nach einem der vorhergehenden Ansprüche, **dadurch gekennzeichnet, dass** man eine Ausgangsfolienbahn verwendet, die im Verlauf ihrer Herstellung einer Querverstreckung unterworfen worden ist.

10. Folienbahn, erhältlich durch ein Verfahren nach einem der vorhergehenden Ansprüche.

11. Folienbahn nach Anspruch 10, mit einer Dicke im Bereich von 15 bis 20 µm.

12. Verwendung der Folienbahn nach Anspruch 10 oder 11 als Hygienefolie oder Medikalfolie.

## Claims

1. A process for the production of a film web, wherein a starting film web of thermoplastic polymer material containing a polyethylene matrix containing 1 to 70 parts by weight of polypropylene, based on 100 parts by weight of polyethylene matrix, is passed, after warming, through a cooled roll nip, **characterized in that** the starting film web is warmed until the molten state of the polyethylene matrix material, but not until the molten state of the polypropylene.

2. The process according to claim 1, **characterized in that** a starting film web containing 5 to 45 parts by weight of polypropylene, based on 100 parts by weight of polyethylene matrix, is used.

3. The process according to claim 2, **characterized in that** a starting film web containing 10 to 40 parts by weight of polypropylene, based on 100 parts by weight of polyethylene matrix, is used.

4. The process according to any of the preceding claims, **characterized in that** a starting film web containing a polyethylene matrix of 60 % by weight of LDPE and 40 % by weight of LLDPE is used.

5. The process according to claim 3, **characterized in that** a starting film web containing a polyethylene matrix of 60 % by weight of LDPE and 40 % by weight of LLDPE-octene with an amount of octene of 5 to 10 % by weight, and an amount of 30 parts by weight of propylene ethylene copolymer containing 3 to 12 % by weight of ethylene as comonomer, based on 100 parts by weight of polyethylene matrix, is used.

6. The process according to any of the preceding claims, **characterized in that** a starting film web having a thickness of from 10 to 30 µm is used.

7. The process according to any of the preceding claims, **characterized in that** the starting film web is warmed by means of one or more heating rolls.

8. The process according to claim 7, **characterized in that** heating rolls having reduced adhesion tendency to the film web with molten polyethylene matrix are used.

9. The process according to any of the preceding claims, **characterized in that** a starting film web which has been subjected during production to transverse stretching is used.

10. A film web obtainable by a process according to any of the preceding claims.

11. The film web according to claim 10, having a thickness in the range of from 15 to 20 µm.

12. Use of a film web according to claim 10 or 11 as hygiene film or medical film.

## Revendications

1. Procédé de fabrication d'une feuille continue, dans lequel une feuille continue de départ en un matériau polymère thermoplastique, comportant une matrice de polyéthylène contenant pour 100 parties en poids de matrice de polyéthylène 1 à 70 parties en poids de polypropylène, est, après son chauffage, envoyée dans l'espace refroidi situé entre les cylindres, **caractérisé en ce qu'**on procède au chauffage de la feuille continue de départ jusqu'à ce que le matériau de matrice de polyéthylène prenne un état fluide à l'état fondu, mais pas jusqu'à l'état de fluidité à l'état fondu du polypropylène.

2. Procédé selon la revendication 1, **caractérisé en ce qu'**on utilise une feuille continue de départ comportant 5 à 45 parties en poids de polypropylène pour 100 parties en poids de matrice de polyéthylène.

3. Procédé selon la revendication 2, **caractérisé en ce qu'**on utilise une feuille continue de départ comportant 10 à 40 parties en poids de polypropylène pour 100 parties en poids de matrice de polyéthylène.

4. Procédé selon l'une des revendications précédentes, **caractérisé en ce qu'**on utilise une feuille continue de départ comportant une matrice de polyéthylène constituée de 60 % en poids de PEBD et de 40 % en poids de PEBDL.

5. Procédé selon la revendication 3, **caractérisé en ce qu'**on utilise une feuille continue de départ comportant une matrice de polyéthylène constituée de 60 % en poids de PEBD et de 40 % en poids de PEBDL-octène, ayant une teneur en octène de 5 à 10 % en poids, et ayant une teneur en un copolymère du propylène et de l'éthylène contenant 3 à 12 % en poids d'éthylène en tant que comonomère, de 30 parties en poids pour 100 parties en poids de la matrice de polyéthylène.

6. Procédé selon l'une des revendications précédentes, **caractérisé en ce qu'**on utilise une feuille continue de départ ayant une épaisseur de 10 à 30 µm.

7. Procédé selon l'une des revendications précédentes, **caractérisé en ce qu'**on procède au chauffage de la feuille continue de départ à l'aide d'un ou plusieurs cylindres chauffants.

8. Procédé selon la revendication 7, **caractérisé en ce qu'**on utilise des cylindres chauffants ayant une tendance réduite à adhérer à la feuille continue comportant une matrice de polyéthylène fluide à l'état fondu.

9. Procédé selon l'une des revendications précédentes, **caractérisé en ce qu'**on utilise une feuille continue de départ qui, au cours de sa fabrication, a été soumise à un étirage transversal.

10. Feuille continue pouvant être obtenue par un procédé selon l'une des revendications précédentes.

11. Feuille continue selon la revendication 10, ayant une épaisseur de 15 à 20 µm.

12. Utilisation de la feuille continue selon la revendication 10 ou 11 en tant que feuille hygiénique ou feuille à usage médical.
